(19) **Europäisches Patentamt / European Patent Office / Office européen des brevets**

(11) **EP 4 763 225 A1**

(12) # EUROPEAN PATENT APPLICATION
published in accordance with Art. 153(4) EPC

(43) Date of publication:
**24.06.2026 Bulletin 2026/26**

(21) Application number: **24911030.5**

(22) Date of filing: **23.12.2024**

(51) International Patent Classification (IPC):
*A61K 45/06* [(2006.01)]   *A61K 39/39* [(2006.01)]
*A61K 33/32* [(2006.01)]   *A61K 33/30* [(2006.01)]
*A61K 33/26* [(2006.01)]   *A61P 35/00* [(2006.01)]

(86) International application number:
**PCT/CN2024/141431**

(87) International publication number:
**WO 2025/140102 (03.07.2025 Gazette 2025/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.12.2023 CN 202311824856**

(71) Applicant: **Novastra Therapeutics Inc.**
**Dover, DE 19901 (US)**

(72) Inventors:
• **GUO, Junling**
  **Suzhou, Jiangsu 215000 (CN)**
• **HE, Yunxiang**
  **Suzhou, Jiangsu 215000 (CN)**
• **HE, Xianglian**
  **Suzhou, Jiangsu 215000 (CN)**

(74) Representative: **Chung, Hoi Kan**
**Mandarin IP Limited**
**7 Cherry Trees**
**Great Shelford**
**Cambridge CB22 5XA (GB)**

(54) **METAL-POLYPHENOL NANO-COATING-WRAPPED TUMOR WHOLE CELL, PREPARATION METHOD THEREFOR, AND USE THEREOF**

(57)    Disclosed are a metal-polyphenol nano-coating-wrapped tumor whole cell, a preparation method therefor, and use thereof. A plant polyphenol in the present invention and manganese ions can be rapidly assembled at room temperature to form a dense coating on the membrane of a tumor cell. The nano-coating wrapping inactivates the tumor cell, ensuring that the vaccine is safe. The nano-coating can prevent any potential tumor antigen from being lost under physiological conditions. The nano-coating is further modified with a lipopolysaccharide, which can promote the endocytosis of the formed whole-cell vaccine by antigen-presenting cells. While forming the structural coating, ions of the metal manganese can stimulate the STING pathway to enhance the anti-tumor effect.

FIG. 3

EP 4 763 225 A1

**Description**

<u>TECHNICAL FIELD</u>

The present disclosure generally relates to the technical field of biotechnology, and in particular, to whole tumor cell encapsulated by metal-polyphenolic nanocoating, a preparation method and application thereof.

<u>BACKGROUND</u>

Immunotherapy is a new type of tumor treatment modality, which may allow immune cells to actively exert their efficacy by activating the host's own immunity. Immunotherapy causes tumor cells to undergo apoptosis by the immune cells directly or indirectly contacting the tumor cells, thus delaying or inhibiting the tumor progression or metastasis.
Tumor vaccines are one of the important immunotherapeutic strategies that may elicit tumor-specific immune stimulation and have been investigated as a potential for cancer treatment. Currently, a plurality of tumor vaccines are based on individual tumor-associated antigens. Modern sequencing technologies have advanced the research of personalized neoantigen vaccines, such approaches may not provide comprehensive coverage of all antigens present in tumor cells. And due to the limitations in immune recognition mechanisms, the effectiveness of the clinical applications of tumor vaccines has remained restricted and may not have been widely used.
Whole tumor cell vaccines, due to the retention of full spectrum of tumor-associated antigens, may break through the limitations of the lack of a wide range of antigens for different types of tumors and overcome the defects of immune escape triggered by the inadequate expression of a single antigen or a specific antigen, thereby achieving tumor treatment or prevention. The main methods for constructing whole tumor cell vaccines include recombination of tumor cell lysates and modification of the intact tumor cells, including genetic modification, cryoengineering, microneedle patch delivery, and macroporous lyophilization. Though these methods have shown potential, they may not fully preserve all potential tumor antigens or consistently elicit strong anti-tumor immune responses, and the preparation process meybe time-consuming and technically demanding, thereby limiting clinical applicability.
The current construction methods for whole tumor cell vaccines, such as Chinese Patent Application No. 201910810918.5, include knocking out specific genes in melanoma cells by gene editing, so that the melanoma cells are mutated during replication to express non-self-antigens that are easy to be recognized by immune cells, and to promote the dendritic cells (DCs) in the co-culture conditions to express more co-stimulatory proteins and significantly increase the immune response in mice. However, gene editing may carry risks of unintended mutations. For example, in Chinese Patent Application No. 202011095900.0, constructed vaccines are obtained by modifying tumor cells by a covalent bonding method, coupling tumor cells and small molecule agonists to enhance tumor cell recognition by immune cells. However, the method introduces chemical modification, imposing stringent requirements on the safety and reactivity of the coupling agent molecules, while the subsequent separation and purification processes incur substantial manufacturing costs. Moreover, small-molecule agonists serving as the primary recognizing antigens may carry a risk of immune tolerance.

<u>SUMMARY</u>

One or more embodiments of the present disclosure provide a method for preparing metal-polyphenol nanocoating-encapsulated whole tumor cells. The method includes: step (a): mixing a plant polyphenol solution and a tumor cell suspension to obtain a first mixture; step (b): adding a manganese ion-containing compound solution to the first mixture, and mixing to obtain a second mixture, wherein a mass ratio of the plant polyphenol to the manganese ion-containing compound is in a range of 1:2 to 1:8; and step (c): adding a buffer solution to the second mixture, mixing, washing, and centrifuging to obtain metal-polyphenol nanocoating (MPN)-encapsulated tumor cells (e.g., MnTA cells).
One or more embodiments of the present disclosure provide MPN -encapsulated tumor cells prepared according to the aforementioned method, a plant polyphenol-manganese ion nanocoating is constructed on a surface of the tumor cells.
In some embodiments, the plant polyphenol is selected from tannic acid, tara tannin (Tara), and (-)-epigallocatechin gallate (EGCG). In certain embodiments, the manganese ion-containing compound is manganese sulfate monohydrate. In some embodiments, the manganese ion-containing compound may be substituted with a zinc ion-containing compound, a ferrous ion-containing compound, or an aluminum ion-containing compound.
In some embodiments, the MPN-encapsulated tumor cells may be further treated with a lipopolysaccharide solution to obtain a lipopolysaccharide-manganese ion-plant polyphenol (LMP) whole tumor cell vaccine.
In certain embodiments, the MPN-encapsulated tumor cells or the LMP whole tumor cell vaccine may be formulated with pharmaceutically acceptable adjuvants or carriers to obtain a pharmaceutically acceptable composition for use in antitumor therapy.

BRIEF DESCRIPTION OF THE DRAWINGS

FIG. 1 is a zeta potential measurement result of the cells obtained at each step according to Example 1 of the present disclosure;

FIG. 2 is a zeta potential measurement result of the cells obtained at each step according to Examples 2 and 3 of the present disclosure;

FIG. 3 is a scanning electron microscope (SEM) image of LMP whole tumor cell vaccine and untreated tumor cells obtained according to Example 1 of the present disclosure;

FIG. 4 is an SEM image of LMP-Tara whole tumor cell vaccine and LMP-EGCG whole tumor cell vaccine obtained according to Examples 2 and 3 of the present disclosure;

FIG. 5 is an X-ray photoelectron spectroscopy (XPS) result of the LMP whole tumor cell vaccine obtained according to Example 1 of the present disclosure;

FIG. 6 is a transmission electron microscope (TEM) image of a cross-section of the LMP whole tumor cell vaccine and the untreated tumor cells obtained according Example 1 of the present disclosure;

FIG. 7 is a fluorescence micrograph of LMP whole tumor cell vaccine and untreated tumor cells, obtained according to Example 1 of the present disclosure, cultured in DMEM for 12 h and stained with Calcein/PI;

FIG. 8 is a statistical result of cell viability of the LMP whole tumor cell vaccine, obtained according to Example 1 of the present disclosure, cultured in DMEM for 12 h;

FIG. 9 is a diagram of a protein concentration of the LMP whole tumor cell vaccine obtained according to Example 1 of the present disclosure;

FIG. 10 is a schematic comparison diagram of the protein concentrations of the LMP whole tumor cell vaccine and the untreated tumor cells obtained according to Example 1 of the present disclosure;

FIG. 11 is a schematic diagram of the result of DC phagocytosis analyzed by flow cytometry of the LMP whole tumor cell vaccine obtained according to Example 1 of the present disclosure and the cells obtained according to Comparative Examples;

FIG. 12 is a confocal microscopy image of DC phagocytosis of the LMP whole tumor cell vaccine obtained according to Example 1 of the present disclosure;

FIG. 13 is an SEM image of LPS-coated tumor cells;

FIG. 14 is a schematic diagram of p65, TBK1, and IRF3 expression in the LMP whole tumor cell vaccine obtained according to Example 1 of the present disclosure and cells in Comparative Examples;

FIG. 15 is a comparison diagram of tumor volume of the animal model after treatment with the LMP whole tumor cell vaccine obtained according to Example 1 of the present disclosure and the cells in Comparative Examples;

FIG. 16 is a schematic diagram of staining of the major organs of the animal model after treatment with the LMP whole tumor cell vaccine obtained according to Example 1 of the present disclosure and cells in Comparative Examples; and

FIG. 17 is a diagram of serum testing of the animal model after treatment with the LMP whole tumor cell vaccine obtained according to Example 1 of the present disclosure and the cells in Comparative Examples.

DETAILED DESCRIPTION OF THE EMBODIMENTS

The present disclosure will now be further illustrated by way of exemplary embodiments and the accompanying drawings.

In certain embodiments of the present disclosure, a method for preparing MPN-encapsulated tumor cells is provided, including:

Step (a): mixing a plant polyphenol solution and a tumor cell suspension to obtain a first mixture;

Step (b): adding a manganese ion-containing compound solution to the first mixture, and mixing to obtain a second mixture, wherein a mass ratio of the plant polyphenol to the manganese ion-containing compound is in a range of 1:2 to 1:8; and

Step (c): adding a buffer solution to the second mixture, mixing, washing, and centrifuging to obtain tumor cells encapsulated with a metal-polyphenol nanocoating (e.g., MnTA cells). The tumor cells encapsulated with a metal-polyphenol nanocoating refers to the MPN-encapsulated tumor cells and the whole tumor cell encapsulated by metal-polyphenolic nanocoating.

In some embodiments, the plant polyphenol may be selected from tannic acid (TA), tara tannin (Tara), and (-)-epigallocatechin gallate (EGCG).

In some embodiments, the manganese ion-containing compound in the step (b) may be selected from manganese sulfate and its hydrates, or manganese chloride and its hydrates.

In some embodiments, the manganese ion-containing compound in the step (b) may be substituted with one of a zinc ion-containing compound, a ferrous ion-containing compound, and an aluminum ion-containing compound.

In some embodiments, after the step (c), the method may further include: mixing the MPN-encapsulated tumor cells (MnTA cells) with a lipopolysaccharide solution, washing, and centrifuging to obtain a lipopolysaccharide-manganese ion-plant polyphenol (LMP) whole tumor cell vaccine.

In some embodiments, the mixing in the step (a), the step (b), and the step (c) may be performed by vortexing.

In some embodiments, the MPN-encapsulated tumor cells may be first mixed with a binder, then mixed with a lipopolysaccharide. The mass ratio of the binder to the plant polyphenol may be in a range of 1:1 to 1:10.

In some embodiments of the present disclosure, the MPN-encapsulated tumor cells prepared according to the aforementioned method are provided, a plant polyphenol-manganese ion nanocoating is constructed on a surface of the tumor cells. While being the components of the nanocoating and serving a structural role, manganese metal ions can also stimulate the stimulator of interferon genes (STING) pathway, thereby enhancing the anti-tumor effect.

In some embodiments, the method for preparing MPN-encapsulated tumor cells includes:

Step (a): mixing a plant polyphenol solution and a tumor cell suspension by vortexing to obtain a first mixture; the plant polyphenol is one of tannic acid, tara tannin, and (-)-epigallocatechin gallate (EGCG).

Step (b): adding a manganese ion-containing compound solution to the first mixture obtained in the step (a), and mixing by vortexing to obtain a second mixture; the mass ratio of the plant polyphenol to the manganese ion-containing compound is in a range of 1:2 to 1:8, the manganese ion-containing compound is manganese sulfate monohydrate, and the manganese ion-containing compound may be substituted with one of a zinc ion-containing compound, a ferrous ion-containing compound, and an aluminum ion-containing compound.

Step (c): adding a buffer solution to the second mixture, mixing, washing, and centrifuging to obtain MPN-encapsulated tumor cells (e.g., MnTA cells).

In some embodiments, LMP tumor cells may be obtained by mixing the MPN-encapsulated tumor cells obtained in step (c) with a lipopolysaccharide solution, washing, and centrifuging. The MPN-encapsulated tumor cells are first mixed with a binder, then mixed with a lipopolysaccharide (the addition amount and concentration of the lipopolysaccharide showed negligible effects on the preparation of LMP tumor cells, thus, no restrictions are specified herein; these parameters can be optimized based on actual situations). The mass ratio of the binder to the plant polyphenol is in a range of 1:1 to 1:10.

A plant polyphenol-manganese ion nanocoating is constructed on a surface of the tumor cells. When lipopolysaccharide is added, a layer of lipopolysaccharide may be further present on the nanocoating. To promote stronger binding or adhesion, the binder may be added, such as polyethyleneimine (PEI) or polylysine (PL).

In some embodiments, the tumor cells are melanoma cells, murine dendritic cell line 2.4 (DC2.4 cells), or Raw264.7 cells.

In some embodiments, the MPN-encapsulated tumor cells are used to prepare a whole tumor cell vaccine. In some embodiments, the application of the MPN-encapsulated tumor cells includes: mixing at least one of the MPN-encapsulated tumor cells or the whole tumor cell vaccine with a pharmaceutically acceptable adjuvant or other ingredients to obtain a pharmaceutically acceptable formulation.

**Example 1**

MnTA nanocoating-encapsulated tumor cells were prepared according to the following steps:

Step 1: collecting B16F10 cells (also referred to as B16F10 melanoma cells) to obtain a single cell suspension ($2 \times 10^6$ cells/mL); adding a TA solution (10 µL, 20 mg/mL) to 200 µL of the single cell suspension and mixing by vortexing for 10 seconds to obtain a mixture of TA-modified cells, hereafter referred to as a TA cell suspension.

Step 2: adding a manganese sulfate monohydrate ($MnSO_4 \cdot H_2O$) solution (50 µL, 10 mg/mL) to the TA cell suspension obtained in step 1, and mixing by vortexing for 10 seconds to obtain a mixture.

Step 3: adding 500 µL of phosphate-buffered saline (PBS, 100 mM, pH=7.4) to the mixture obtained in step 2; mixing by vortexing for 10 seconds and centrifuging ($500 \times g$, 5 min) to separate the cells to obtain separated cells; washing the separated cells, adding a phosphate buffered saline (PBS) solution (1 mL, 100 mM, pH=7.4) to the separated cells to disperse the cells uniformly, and centrifuging ($500 \times g$, 5 min) to remove the supernatant. This washing procedure was repeated three times to remove excess TA and $Mn^{2+}$ ions. After the final washing, $Mn^{2+}$ and TA nanocoating-modified B16F10 melanoma cells were obtained by centrifugation, hereafter referred to as MnTA cells.

Step 4: suspending the MnTA cells obtained in step 3 in 1 mL of binder (PEI solution, 0.1 mg/mL), mixing by vortexing for 10 seconds to obtain PEI-MnTA cells, adding a lipopolysaccharide solution, and mixing by vortexing for 15 seconds; washing by PBS (100 mM, 7.4) and centrifuging to obtain LMP cells (also referred to as LMP whole tumor cell vaccine).

**Example 2**

MnTara nanocoating-encapsulated tumor cells were prepared according to the following steps:

Step 1: collecting B16F10 cells to obtain a single cell suspension ($2 \times 10^6$ cells/mL); adding a Tara solution (10 $\mu$L, 20 mg/mL) to 200 $\mu$L of the single cell suspension and mixing by vortexing for 10 seconds to obtain a mixture of Tara-modified cells, hereafter referred to as a Tara cell suspension.
Step 2: adding a manganese sulfate monohydrate ($MnSO_4 \cdot H_2O$) solution (50 $\mu$L, 10 mg/mL) to the Tara cell suspension obtained in step 1, and mixing by vortexing for 10 seconds to obtain a mixture.
Step 3: adding 500 $\mu$L of PBS solution (100 mM, pH=7.4) to the mixture obtained in step 2; mixing by vortexing for 10 seconds and centrifuging ($500 \times$ g, 5 min) to separate the cells to obtain separated cells; washing the separated cells, adding a PBS solution (1 mL, 100 mM, pH=7.4) to the separated cells to disperse the cells uniformly, and centrifuging ($500 \times$g, 5 min) to remove the supernatant. This washing procedure was repeated three times to remove excess Tara and $Mn^{2+}$ ions. After the final washing, $Mn^{2+}$ and Tara nanocoating-modified B16F10 melanoma cells were obtained by centrifugation, hereafter referred to as MnTara cells.
Step 4: suspending the MnTara cells obtained in step 3 in 1 mL of PEI solution (0.1 mg/mL), mixing by vortexing for 10 seconds to obtain PEI-MnTara cells, adding a lipopolysaccharide solution, and mixing by vortexing for 15 seconds; washing by PBS (100 mM, 7.4) and centrifuging to obtain LMP-Tara cells (also referred to as LMP-Tara whole tumor cell vaccine).

**Example 3**

MnEGCG nanocoating-encapsulated tumor cells were prepared according to the following steps:

Step 1: collecting B16F10 cells to obtain a single cell suspension ($2 \times 10^6$ cells/mL); adding a EGCG solution (10 $\mu$L, 20 mg/mL) to 200 $\mu$L of the single cell suspension and mixing by vortexing for 10 seconds to obtain a mixture of EGCG-modified cells, hereafter referred to as a EGCG cell suspension.
Step 2: adding a $MnSO_4 \cdot H_2O$ solution (50 $\mu$L, 10 mg/mL) to the EGCG cell suspension obtained in step 1, and mixing by vortexing for 10 seconds to obtain a mixture.
Step 3: adding 500 $\mu$L of PBS solution (100 mM, pH=7.4) to the mixture obtained in step 2; mixing by vortexing for 10 seconds and centrifuging ($500 \times$ g, 5 min) to separate the cells to obtain separated cells; washing the separated cells, adding a PBS solution (1 mL, 100 mM, pH=7.4) to the separated cells to disperse the cells uniformly, and centrifuging ($500 \times$g, 5 min) to remove the supernatant. This washing procedure was repeated three times to remove excess EGCG and $Mn^{2+}$ ions. After the final washing, $Mn^{2+}$ and EGCG nanocoating-modified B16F10 melanoma cells were obtained by centrifugation, hereafter referred to as MnEGCG cells.
Step 4: suspending the MnEGCG cells obtained in step 3 in 1 mL of PEI solution (0.1 mg/mL), mixing by vortexing for 10 seconds to obtain PEI-MnEGCG cells, adding a lipopolysaccharide solution, and mixing by vortexing for 15 seconds; washing by PBS (100 mM, 7.4) and centrifuging to obtain LMP-MGCG cells (also referred to as LMP-EGCG whole tumor cell vaccine).

**Example 4**

MnTA nanocoating-encapsulated tumor cells were prepared according to the following steps:

Step 1: collecting DC2.4 cells to obtain a single cell suspension ($2 \times 10^6$ cells/mL); adding a TA solution (10 $\mu$L, 20 mg/mL) to 200 $\mu$L of the single cell suspension and mixing by vortexing for 10 seconds to obtain a mixture of TA-modified cells, hereafter referred to as a TA cell suspension.
Step 2: adding a $MnSO_4 \cdot H_2O$ solution (50 $\mu$L, 10 mg/mL) to the TA cell suspension obtained in step 1, and mixing by vortexing for 10 seconds to obtain a mixture.
Step 3: adding 500 $\mu$L of PBS solution (100 mM, pH=7.4) to the mixture obtained in step 2; mixing by vortexing for 10 seconds and centrifuging ($500 \times$ g, 5 min) to separate the cells to obtain separated cells; washing the separated cells, adding a PBS solution (1 mL, 100 mM, pH=7.4) to the separated cells to disperse the cells uniformly, and centrifuging ($500 \times$g, 5 min) to remove the supernatant. This washing procedure was repeated three times to remove excess TA and $Mn^{2+}$ ions. After the final washing, $Mn^{2+}$ and TA nanocoating-modified DC2.4 cells were obtained by centrifugation, hereafter referred to as MnTA cells.

Step 4: suspending the MnTA cells obtained in step 3 in 1 mL of PEI solution (0.1 mg/mL), mixing by vortexing for 10 seconds to obtain PEI-MnTA cells, adding a lipopolysaccharide solution, and mixing by vortexing for 15 seconds; washing by PBS (100 mM, 7.4) and centrifuging to obtain coating-modified cells.

**Example 5**

MnTA nanocoating-encapsulated tumor cells were prepared according to the following steps:

Step 1: collecting Raw264.7 cells to obtain a single cell suspension ($2 \times 10^6$ cells/mL); adding a TA solution (10 $\mu$L, 20 mg/mL) to 200 $\mu$L of the single cell suspension and mixing by vortexing for 10 seconds to obtain a mixture of TA-modified cells, hereafter referred to as a TA cell suspension.

Step 2: adding a $MnSO_4 \cdot H_2O$ solution (50 $\mu$L, 10 mg/mL) to the TA cell suspension obtained in step 1, and mixing by vortexing for 10 seconds to obtain a mixture.

Step 3: adding 500 $\mu$L of PBS solution (100 mM, pH=7.4) to the mixture obtained in step 2; mixing by vortexing for 10 seconds and centrifuging ($500 \times g$, 5 min) to separate the cells to obtain separated cells; washing the separated cells, adding a PBS solution (1 mL, 100 mM, pH=7.4) to the separated cells to disperse the cells uniformly, and centrifuging ($500 \times g$, 5 min) to remove the supernatant. This washing procedure was repeated three times to remove excess TA and $Mn^{2+}$ ions. After the final washing, $Mn^{2+}$ and TA nanocoating-modified Raw264.7 cells were obtained by centrifugation, hereafter referred to as MnTA cells.

Step 4: suspending the MnTA cells obtained in step 3 in 1 mL of PEI solution (0.1 mg/mL), mixing by vortexing for 10 seconds to obtain PEI-MnTA cells, adding a lipopolysaccharide solution, and mixing by vortexing for 15 seconds; washing by PBS (100 mM, 7.4) and centrifuging to obtain coating-modified cells.

**Example 6**

MnTA nanocoating-encapsulated tumor cells were prepared according to the following steps:

Step 1: collecting B16F10 cells to obtain a single cell suspension ($2 \times 10^6$ cells/mL); adding a TA solution (10 $\mu$L, 20 mg/mL) to 200 $\mu$L of the single cell suspension and mixing by vortexing for 10 seconds to obtain a mixture of TA-modified cells, hereafter referred to as a TA cell suspension.

Step 2: adding a $MnSO_4 \cdot H_2O$ solution (40 $\mu$L, 10 mg/mL) to the TA cell suspension obtained in step 1, and mixing by vortexing for 10 seconds to obtain a mixture.

Step 3: adding 500 $\mu$L of PBS solution (100 mM, pH=7.4) to the mixture obtained in step 2; mixing by vortexing for 10 seconds and centrifuging ($500 \times g$, 5 min) to separate the cells to obtain separated cells; washing the separated cells, adding a PBS solution (1 mL, 100 mM, pH=7.4) to the separated cells to disperse the cells uniformly, and centrifuging ($500 \times g$, 5 min) to remove the supernatant. This washing procedure was repeated three times to remove excess TA and $Mn^{2+}$ ions. After the final washing, $Mn^{2+}$ and TA nanocoating-modified B16F10 melanoma cells were obtained by centrifugation, hereafter referred to as MnTA cells.

Step 4: suspending the MnTA cells obtained in step 3 in 1 mL of PEI solution (0.02 mg/mL), mixing by vortexing for 10 seconds to obtain PEI-MnTA cells, adding a lipopolysaccharide solution, and mixing by vortexing for 15 seconds; washing by PBS (100 mM, 7.4) and centrifuging to obtain LMP cells (also referred to as LMP whole tumor cell vaccine).

**Example 7**

MnTA nanocoating-encapsulated tumor cells were prepared according to the following steps:

Step 1: collecting B16F10 cells to obtain a single cell suspension ($2 \times 10^6$ cells/mL); adding a TA solution (10 $\mu$L, 20 mg/mL) to 200 $\mu$L of the single cell suspension and mixing by vortexing for 10 seconds to obtain a mixture of TA-modified cells, hereafter referred to as a TA cell suspension.

Step 2: adding a $MnSO_4 \cdot H_2O$ solution (160 $\mu$L, 10 mg/mL) to the TA cell suspension obtained in step 1, and mixing by vortexing for 10 seconds to obtain a mixture.

Step 3: adding 500 $\mu$L of PBS solution (100 mM, pH=7.4) to the mixture obtained in step 2; mixing by vortexing for 10 seconds and centrifuging ($500 \times g$, 5 min) to separate the cells to obtain separated cells; washing the separated cells, adding a PBS solution (1 mL, 100 mM, pH=7.4) to the separated cells to disperse the cells uniformly, and centrifuging ($500 \times g$, 5 min) to remove the supernatant. This washing procedure was repeated three times to remove excess TA and $Mn^{2+}$ ions. After the final washing, $Mn^{2+}$ and TA nanocoating-modified B16F10 melanoma cells were obtained by centrifugation, hereafter referred to as MnTA cells.

Step 4: suspending the MnTA cells obtained in step 3 in 1 mL of PEI solution (0.2 mg/mL), mixing by vortexing for 10 seconds to obtain PEI-MnTA cells, adding a lipopolysaccharide solution, and mixing by vortexing for 15 seconds; washing by PBS (100 mM, 7.4) and centrifuging to obtain LMP cells (also referred to as LMP whole tumor cell vaccine).

In the following tests and experiments, TA cells are B16F10 melanoma cells modified with TA, MnTA cells are B16F10 melanoma cells modified with $Mn^{2+}$ and TA nanocoating, and LMP whole tumor cell vaccine is LPS-modified MnTA cells.

The cell culture conditions in the following embodiments are as follows: B16F10 cells, DC2.4 cells, TA cells, MnTA cells, LMP whole tumor cell vaccine, and other cells are incubated in DMEM medium (DMEM, Sigma, USA) containing 10 % fetal bovine serum (FBS, Gibco) and 1 % penicillin-streptomycin at 37 °C with a $CO_2$ concentration of 5%. Solutions in one or more embodiments in the present disclosure are freshly prepared and filtered through a 0.2 micron filter membrane for immediate use.

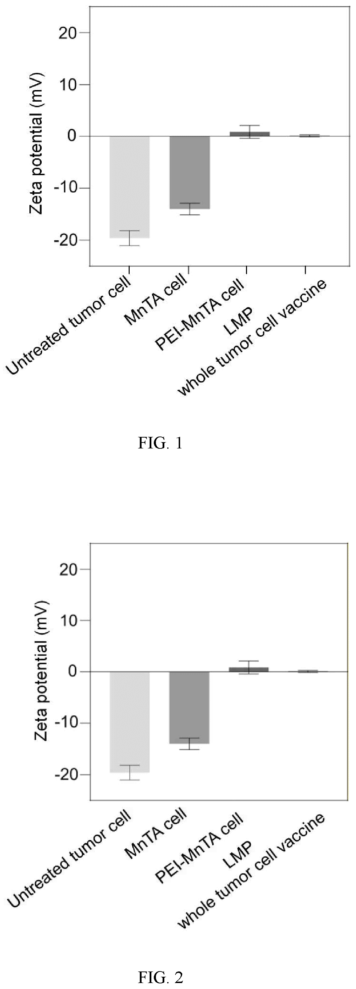

FIG. 1 is a zeta potential measurement result of the cells obtained at each step according to Example 1 of the present disclosure. As shown in FIG. 1, after the formation of the nanocoating composed of $Mn^{2+}$ and TA on the surface of untreated tumor cells to form the MnTA cells, the zeta potential value of the MnTA cells was shifted positively. This is attributed to the $Mn^{2+}$/TA nanocoating formed on the surface of untreated tumor cells, which alters the native negative charge of the cell membrane. The zeta potential value of the MnTA cells confirms the successful formation of the $Mn^{2+}$/TA nanocoating. The zeta potential value of the prepared LMP whole tumor cell vaccine was shifted slightly positively (1.30 eV) compared with that of the PEI-MnTA cells, indicating the successful preparation of the LMP whole tumor cell vaccine.

FIG. 2 is a zeta potential measurement result of the cells obtained at each step according to Examples 2 and 3 of the present disclosure. As shown in FIG. 2, the result is similar to that in Example 1.

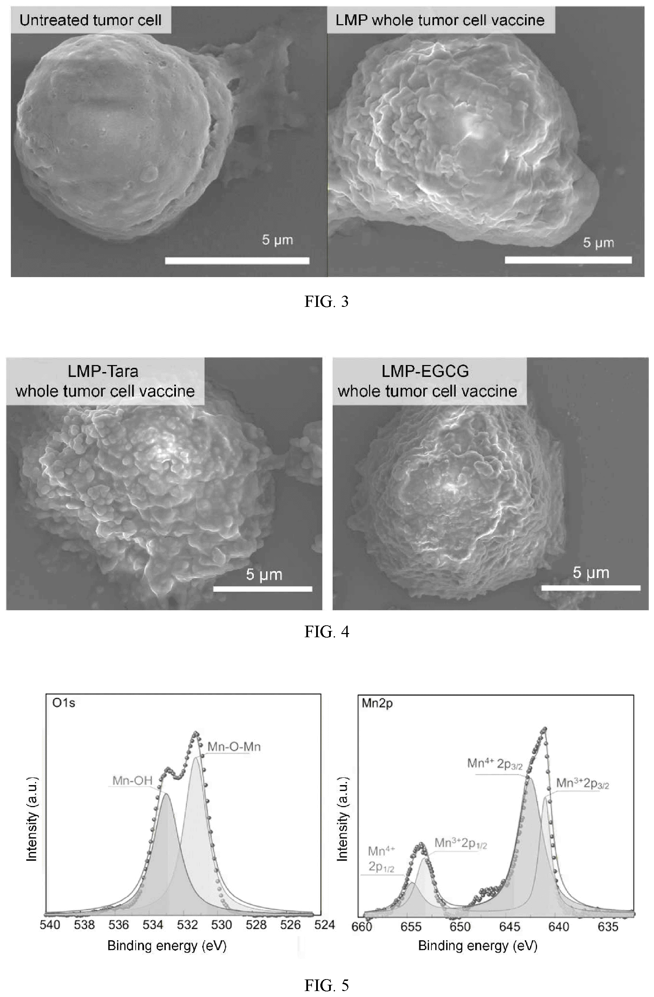

FIG. 3 is a SEM image of LMP whole tumor cell vaccine and untreated tumor cells obtained according to Example 1 of the present disclosure. FIG. 4 is an SEM image of LMP-Tara whole tumor cell vaccine and LMP-EGCG whole tumor cell vaccine obtained according to Examples 2 and 3 of the present disclosure. As shown in FIG. 3, the LMP whole tumor cell vaccine has the similar size as the untreated tumor cells, but with a rougher surface. As shown in Fig. 4, the use of Tara and EGCG also roughened the surface of the encapsulated cells (Fig. 4), indicating that Tara and EGCG can also achieve the formation of a metal-polyphenol network with $Mn^{2+}$ to modify individual cells.

FIG. 5 is an XPS result of the LMP whole tumor cell vaccine obtained according to Example 1 of the present disclosure. As shown in FIG. 5, the O 1s spectrum shows the formation of Mn-O-Mn and Mn-O bonds, indicating that $Mn^{2+}$ has undergone complexation with TA and has formed a nanocoating; the Mn 2p spectrum shows that the peak of Mn has split, and peaks appear at 643 eV and 645 eV, indicating that $Mn^{2+}$ has undergone complexation with TA.

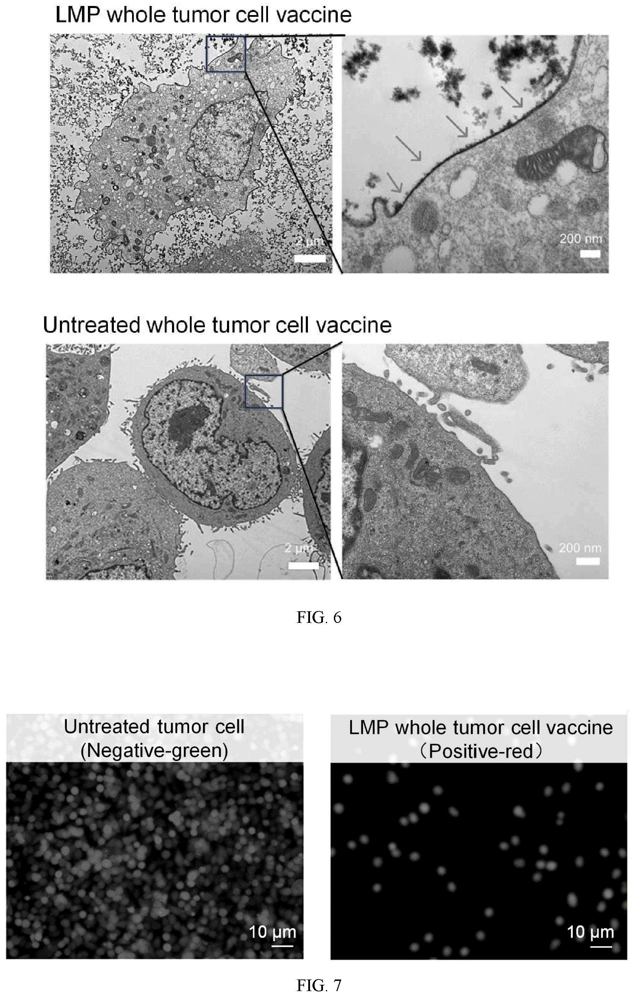

FIG. 6 is a TEM image of a cross-section of the LMP whole tumor cell vaccine and the untreated tumor cells obtained according Example 1 of the present disclosure. As shown in FIG. 6, a continuous nanocoating is formed on the cell membrane of the LMP whole tumor cell vaccine, whereas smooth cell membranes are seen on the untreated tumor cells, indicating that the nanocoating formed by the $Mn^{2+}$ and TA completely encapsulated the tumor cells.

To construct a whole tumor cell vaccine, the following three critical aspects need to be considered:

(1) Whether tumor cells can be effectively inactivated. Vaccines constructed based on tumor cells have the potential to induce new tumors in the host after injection because they retain all tumor-associated antigens. Therefore, effective inactivation of the whole tumor cell vaccine constitutes the first critical step for determining its clinical applicability tumor cell vaccine.

(2) Whether all tumor cell antigens can be effectively retained. To overcome the limitation of immune escape caused by insufficient expression of a single antigen or specific antigen, and to achieve effective treatment of tumors, it is necessary to retain as many tumor cell antigens as possible.

(3) Equipped with an adjuvant such as an antigen-presenting cell activator (e.g., interferon). Activation of the immune system by the whole tumor cell vaccine is directly dependent on the sustained activation of antigen-presenting cells (e.g., the DCs) associated with innate immune stimulation. In addition to direct cytotoxicity against tumor cells, activators may promote DC maturation and antigen presentation, thereby bridging the innate immune response and the adaptive immune response; the whole tumor cell vaccine in the present disclosure circumvents such limitations.

Safety evaluation of the LMP whole tumor cell vaccine:

I. Staining-based assessment of cell viability: the MnTA nanocoating-modified B16F10 cells in Example 1 were incubated with DMEM medium (10 % serum, 1 % penicillin-streptomycin) in a cell culture incubator (37 °C, 5 % $CO_2$) for varying durations up to 12 h. The cells were then collected, wash with PBS, and resuspended with 195 $\mu$L of Annexin V-fluorescein isothiocyanate (Annexin V-FITC) conjugate solution together with 5 $\mu$L of propidium iodide (PI) solution per $5 \times 10^4$ cells. The cells were incubated at 37 °C in dark for 20 min and analyzed by flow cytometry.

II. Cell Counting Kit-8 (CCK-8) assay: simply incubate the cells (B16F10 cells, LMP whole tumor cell vaccine) at a density of $1.0 \times 10^5$ cells/mL for 24 h in an opaque white 96-well plate. Subsequently, add 10 $\mu$L of CCK8 solution to each well, and detect the absorbance at 450 nm using a microplate reader (enzyme-linked immunosorbent assay plate reader) (microtiter reader, Infinite® 200 PRO, Tecan, Switzerland) after incubation for 1 h in a cell culture incubator. Add CCK-8 reagent to each well, and then incubate for 60 min and measure the absorbance.

Untreated B16F10 cells are used as a control group, and all experiments are repeated three times independently.

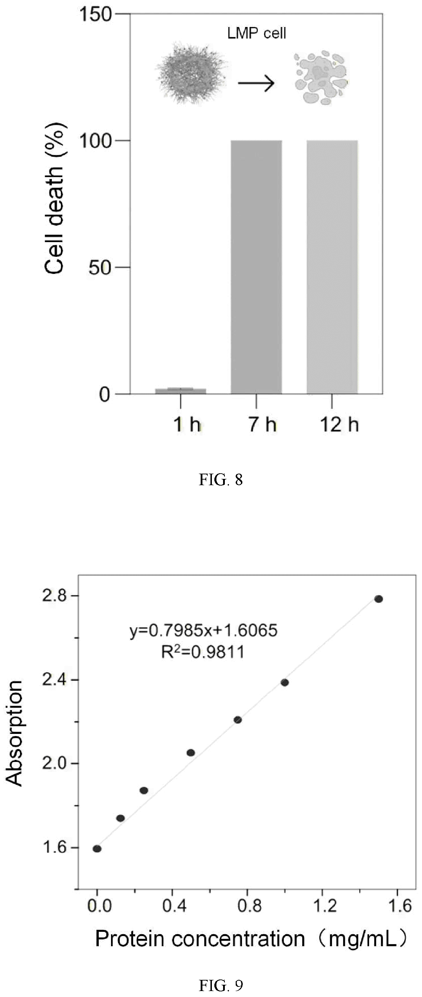

To verify the survival of the LMP whole tumor cell vaccine, necrosis-dependent cell death was confirmed by the apoptosis kit staining (Annexin V-FITC/PI). FIG. 7 is a fluorescence micrograph of LMP whole tumor cell vaccine and untreated tumor cells, obtained according to Example 1 of the present disclosure, cultured in DMEM for 12 h and stained with Calcein/PI. As shown in FIG. 7, almost all of the LMP whole tumor cell vaccine are positive, indicating that the cells are died. FIG. 8 is a statistical result of cell viability of the LMP whole tumor cell vaccine, obtained according to Example 1 of the present disclosure, cultured in DMEM for 12 h. As shown in FIG. 8, the LMP whole tumor cell vaccine does not show proliferative activity in the Cell Counting Kit-8 (CCK8) assay compared to untreated tumor cells. By incubating the LMP whole tumor cell vaccine in the medium after less than 12 h, the LMP whole tumor cell vaccine completely lost their ability to proliferate. As shown in the test result, the tumor cell-based LMP whole tumor cell vaccine is non-tumorigenic, eliminating the possibility of unintended secondary tumorigenesis.

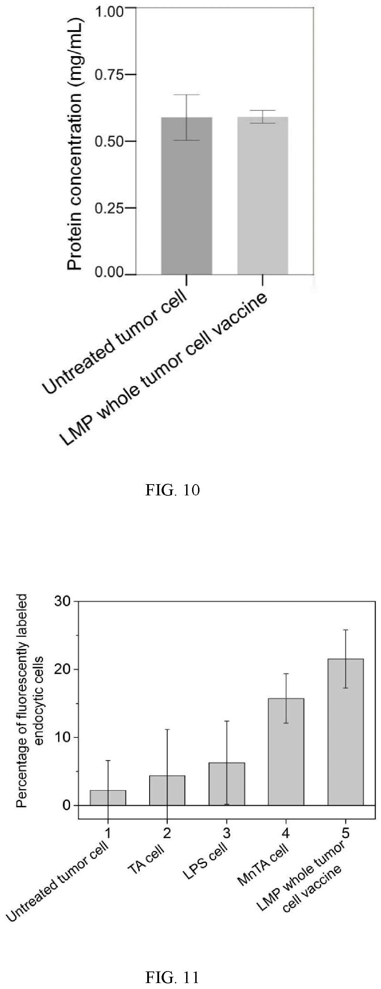

FIG. 9 is a diagram of a protein concentration of the LMP whole tumor cell vaccine obtained according to Example 1 of the present disclosure. FIG. 10 is a schematic comparison diagram of the protein concentrations of the LMP whole tumor cell vaccine and the untreated tumor cells obtained according to Example 1 of the present disclosure. The same number of LMP whole tumor cell vaccine and untreated tumor cells were suspended in PBS. Protein concentrations were determined using the Bradford Protein Assay Kit according to the manufacturer's instructions. Protein concentrations were determined based on the standard curve and sample volume, and the results are shown in FIG. 9 and FIG. 10. As shown in FIGs. 9-10, the protein concentration of untreated tumor cells is $591.4 \pm 24$ $\mu$g-mL$^{-1}$ ($1182.8 \pm 4.8$ pg per cell), whereas that of LMP whole tumor cell vaccine is $589.2 \pm 85$ $\mu$g-mL$^{-1}$ ($1178.4 \pm 17.8$ pg per vaccine). Additionally, there is no significant difference in the RNA concentration between the untreated tumor cells and the LMP whole tumor cell vaccine. More than 99% of the potential tumor antigens are encapsulated by the MnTA nanocoating, indicating that the nanocoating formed by Mn$^{2+}$ and TA retains all the tumor antigens and facilitates immune stimulation.

Evaluation of LPS coating for enhanced uptake and activation of DCs

The uptake of antigens by antigen-presenting cells such as DCs is an important parameter in evaluating the effectiveness of a vaccine. After phagocytosis of tumor antigens, immature DCs are further changed into mature DCs by secreting pro-inflammatory factors, including tumor necrosis factor-alpha (TNF-$\alpha$) and interleukin-6 (IL-6). Mature DCs have higher expressions of CD80 and CD86 factors.

To assess the role of LPS coating, bone marrow-derived dendritic cells (BMDCs) are co-incubated with the LMP whole tumor cell vaccine. The LMP whole tumor cell vaccine is first stained with a far-infrared fluorescent cell membrane probe (1,1'-dioctadecyl-3,3,3',3'-tetramethylindodicarbocyanine, DiD). The phagocytic (uptake) capacity of DCs for the LMP whole tumor cell vaccine was observed by confocal laser scanning microscopy (CLSM, Leica TCS SP8). Label the B16F10 cells with DiD, denoted as DiD-B16F10, by following process: diluting DiD to a 10 $\mu$M DiD solution, mixing with the B16F10 cells, and staining for 20 min. DiD-labeled B16F10 cells were then processed into DiD-labeled LMP whole tumor cell vaccine (DiD-LMP vaccine) according to the method described in Example 1.

BMDCs were extracted by following process: killing the mice by neck-breaking, immersing in 75% alcohol for 2 min, taking out the mice, and cutting off the humerus and tibia of the mice; soaking the removed humerus and tibia in 75 % alcohol for 2 min after removing residual tissue, and then placing in PBS (pH=7.4); cutting off the ends of the humerus and tibia, and flushing the BMDCs from the ends of the humerus and tibia sequentially using a sterile syringe filled with PBS; collecting bone marrow cell suspensions, centrifuging (1500 rpm, 6 min) to remove PBS to obtain the bone marrow cells, culturing in six-well plates with Roswell Park Memorial Institute 1640 (RPMI-1640 medium) containing granulocyte-macrophage colony-stimulating factor (GM-CSF, 10 ng/mL) and interleukin-4 (IL-4, 5 ng/mL) to induce differentiation of the bone marrow cells. Finally, the bone marrow cells were placed in an incubator (37°C, 5% CO$_2$) and the time of extraction was recorded as day 0. The medium on the third day was changed to continue the incubation, and on day 5, the suspended cells were colleced as immature BMDCs cells.

After DC 2.4 was incubated in a confocal dish for 24 h, add DiD-LMP vaccine and co-incubation with DC 2.4 for 6 h. Wash the cells with PBS, fix the cells with 4 % paraformaldehyde for 15 min, and then place the cells at 4 °C for 12 h. Wash the cells with PBS, permeabilize the cells with 0.1 % Triton-X in PBS for 15 min, and block with 1 % bovine serum albumin (BSA) for 20 min, and then label the cells with phalloidin-fluorescein isothiocyanate ( phalloidin-FITC) in 1 % BSA for 1h. After a final wash in PBS, DCs were stained with 4',6-Diamidino-2-Phenylindole (DAPI). The cells were analyzed by laser confocal microscope observation. Detection using flow cytometry included the following process: co-culturing the DiD-LMP vaccine with DC2.4 for 6 h, and collecting the cells and performing fluorometric analysis directly in a flow cytometer.

Activation of DCs by LMP whole tumor cell vaccine: incubating immature BMDCs overnight, and co-culturing DiD-B16F10, DiD-MnTA-modified B16F10 cells, DiD-LMP vaccine, and DiD-TA-modified B16F10 cells with BMDCs for 24 h (37°C, 5% CO$_2$) respectively; co-incubating the cells with FITC-CD11c for 15 min, and performing fluorescence analysis by flow cytometry. DiD-B16F10, DiD-MnTA-modified B16F10 cells, and DiD-TA-modified B16F10 cells were used as Comparative Examples. To illustrate whether LPS has a synergistic effect with the metal-polyphenol coating, LPS cells were obtained by wrapping LPS with tumor cells. The tumor cells are mixed by vortexing with the LPS solution for 10 s to obtain LPS cells, and the LPS cells are used as a Comparative Example.

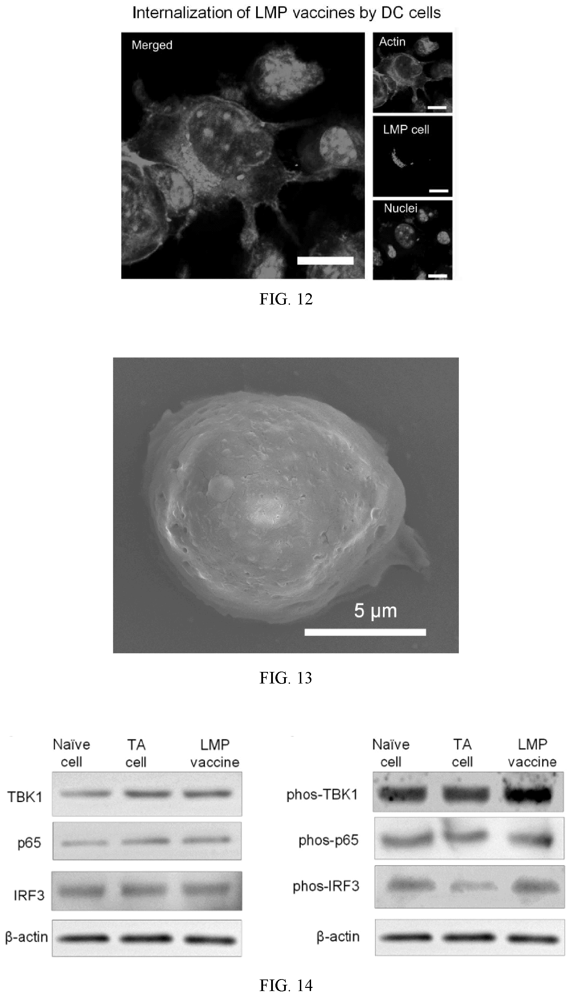

FIG. 11 is a schematic diagram of the result of DC phagocytosis analyzed by flow cytometry of the LMP whole tumor cell vaccine obtained according to Example 1 of the present disclosure and the cells obtained according to Comparative Examples. FIG. 12 is a confocal microscopy image of DC phagocytosis of the LMP whole tumor cell vaccine obtained according to Example 1 of the present disclosure. FIG. 13 is an SEM image of LPS-coated tumor cells. As shown in FIG. 11, compared to MnTA cells, the LMP whole tumor cell vaccine exhibited a twofold enhancement in cellular internalization, indicating that LPS functionalization plays a key role in the LMP whole tumor cell vaccine. Compared to cells modified with LPS alone, the LMP whole tumor cell vaccine exhibited a three-fold enhancement in cellular internalization, indicating that the combination of LPS with TA-Mn is essential for promoting DC phagocytosis of the LMP whole tumor cell vaccine. As shown in FIG. 12, the CLSM confirmed the internalization of the LMP whole tumor cell vaccine labeled by DiD (red) by DCs within a co-incubation of LMP whole tumor cell vaccine and BMDCs, indicating that the increased uptake of the LMP whole tumor cell vaccine is attributed to LPS. Tumor cells encapsulated with pure LPS showed insignificant fluorescence intensity, indicating that LPS is unable to adhere directly on the cell surface for encapsulation and therefore internalization was not effective. As shown in FIG. 13, the surface of the LPS-treated cells, which still remained smooth, is due to the presence of a small amount of LPS, and therefore led to the low internalization efficiency of the DC cells.

STING signaling activation experiment in vitro

The experiment included the following process:
inoculating the BMDCs in 6-well plates at a density of $1 \times 10^6$ cells per well, culturing overnight, and incubating the LMP whole tumor cell vaccine with BMDCs for 24 h; using trypsin digestion to detach a portion of the cells, and collecting and suspending the cells in PBS; extracting protein samples from BMDCs and dispersing in PBS; and determining a protein concentration using bicinchoninic acid (BCA) assay; mixing the extracted proteins with protein uploading buffer solution and denaturing the proteins by placing them in a boiling water bath for 5 min; adding denatured proteins and markers to the gel wells for electrophoresis and finally staining to analyze the expression of TBK1, p-TBK1, NF-κB p65 subunit (RelA)-p65, p-p65, IRF3, and pIRF3; collecting the other portion of the cells to extract total RNA with Triquick extraction reagent, and subsequently, quantifing the RNA (500 ng) with NanoDrop2000, following by reverse transcription of the extracted RNA to cDNA; and performing Real-time fluorescent quantitative PCR using SYBR green dye on quant studio 3 applied biosystems.

Primer sequences are shown in Table 1.

PCR experiment parameters included initial denaturation at 95°C for 30 s, denaturation at 95°C for 10 s, 40 cycles, and annealing the primers at 60°C for 30 s. Comparative quantitative calculations are performed using the 2-ΔΔCt manner with glyceraldehyde 3-phosphate dehydrogenase (GAPDH) as an endogenous control.

Table 1. Primer sequences for RT-qPCR

| substrate | primer sequence | |
| --- | --- | --- |
|  | Prelude (5'-3') | Reverse (5'-3') |
| ACSL4 | TGAACGTATCCCTGGACTAGG | TCAGACAGTGTAAGGGGTGAA |
| PTGS2 | TGAGCAACTATTCCAAACCAGC | AGTCCGGGTACAGTCACACTT |
| GPX4 | GCCTGGATAAGTACAGGGGTT | CATGCAGATCGACTAGCTGAG |

Proteins that may be associated with the STING pathway were first evaluated in BMDCs. BMDCs were treated and co-incubated with untreated tumor cells, TA-encapsulated tumor cells (TA cells, prepared according to Example 1), and LMP whole tumor cell vaccine, respectively. The LMP whole tumor cell vaccine is most significant for STING pathway-associated phosphorylation of p65, TBK1 and IRF3 (phos-p65, phos-TBKI and phos-IRF3). FIG. 14 is a schematic diagram of p65, TBK1, and IRF3 expression in the LMP whole tumor cell vaccine obtained according to Example 1 of the present disclosure and cells in Comparative Examples. As shown in FIG. 14, the left panel shows the results of gel electrophoresis analysis for p65, TBK1, and IRF3; and the right panel shows the phos-p65, phos-TBK1, and phos-IRF3 expression results.

The research on in vivo treatment using the LMP whole tumor cell vaccine obtained according to one or more embodiments of the present disclosure included the following process:

using subcutaneous melanoma mice as a model; the melanoma mice model was established as follows (the animal model in the accompanying illustration is this melanoma mice model):
collecting healthy B16F10 cells and resuspending in PBS with a cell density of $6\times10^6$ cells/mL; injecting 100 $\mu$L into the back above the right leg of four-week-old C57BL/6 mice to establish a mouse subcutaneous melanoma model, which is recorded as day 0; the injected mice was used for subsequent experiments, and the size of the tumors was detected with vernier calipers.

Combining cell therapy of LMP whole tumor cell vaccine and immunotherapy (loaded with PD-L1) for synergy therapy; the process of the synergy therapy included:

randomly dividing 32 C57BL/6 mice with tumor into four groups (n=8), including PBS group, LMP whole tumor cell vaccine group, TA vaccine group, and LMP+anti-PD-L1 group. Anti-PD-L1 was administered by tail vein injection on days 3, 6, 9, and 14 (anti-PD-L1: 7.5 mg/kg). The LMP whole tumor cell vaccine group and the TA vaccine group were injected with LMP whole tumor cell vaccine ($3\times10^5$ cells/pupil) or TA whole tumor cell vaccine by abdominal administration on days 4, 7, 10, and 15 respectively. The LMP+anti-Pd-L1 group was administered anti-PD-L1 (7.5 mg/kg) by intravenous injection every three days starting on the third day, and LMP whole tumor cell vaccine ($3\times10^5$ cells/vessel) was administered by abdominal injection every day starting on the fourth day for a total of four administrations. The mice were weighed every two days during the treatment period, and the sizes of the tumors were measured with vernier calipers until day 24. Tumor volume is determined according to the following formula:

$$V\ (\text{mm}^3) = L \times W^2 / 2$$

where V is the tumor volume, L is the length of the tumor, and W is the width of the tumor.

The experimental results are as follows:

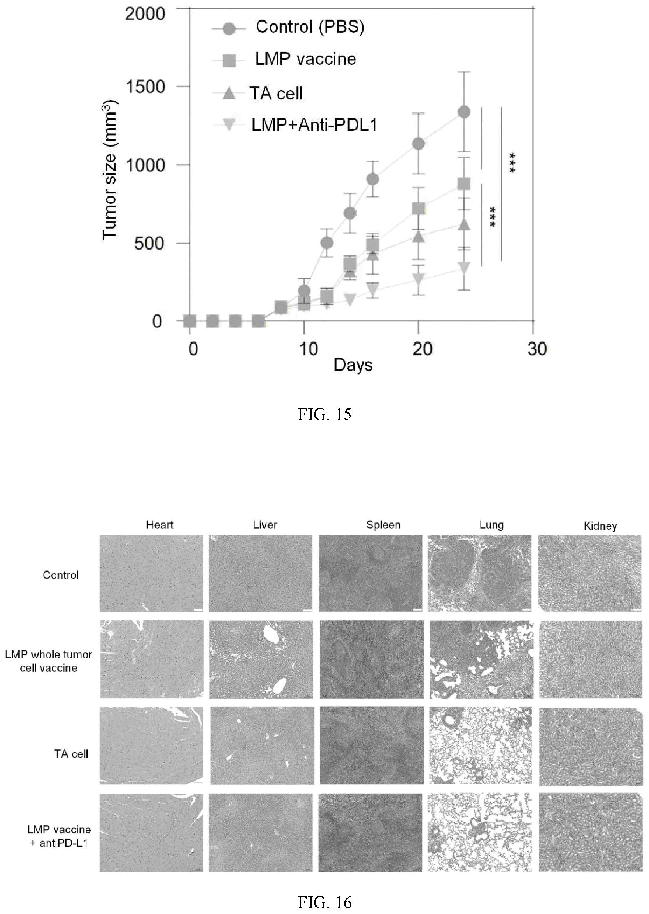

FIG. 15 is a comparison diagram of tumor volume of the animal model after treatment with the LMP whole tumor cell vaccine obtained according to Example 1 of the present disclosure and the cells in Comparative Examples. As shown in FIG.15, the LMP whole tumor cell vaccine significantly inhibited tumor growth compared to the PBS group, while the TA vaccine group exhibited a moderate growth inhibitory effect, demonstrating the critical role of MnTA nano camouflage in potentiating anti-tumor immunotherapy. The addition of anti-PD-L1 antibodies to the treatment model achieved further enhanced anti-tumor efficacy. Notably, all mice in the combination therapy group exhibited minimal tumor volumes, which were significantly reduced after treatment. The data demonstrate that the whole tumor cell vaccine proposed in the present disclosure, when used in combination with immunotherapy, effectively inhibits tumor growth, confirming its potential for therapeutic applications in oncology.

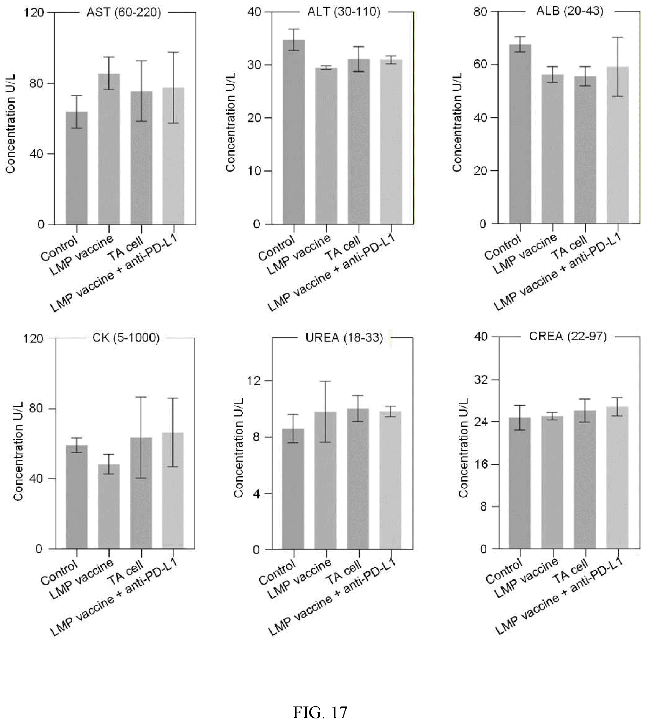

After the above in vivo experiments, the mice were executed, major organs (e.g., heart, liver, spleen, lungs, and kidneys) were isolated, and tumors were collected, fixed in 4% paraformaldehyde, and examined pathologically. The results of histologic sections of major organs (e.g., heart, liver, spleen, and kidneys) for pathologic analysis (hemoglobin and eosinophilic staining, H&E staining) are shown in FIG. 16. FIG. 16 is a schematic diagram of staining of the major organs of the animal model after treatment with the LMP whole tumor cell vaccine obtained according to Example 1 of the present disclosure and cells in Comparative Examples. As shown in FIG. 16, there is no significant difference in the mice after the different treatments. A comprehensive toxicity assessment of key blood biochemical analysis parameters including aspartate aminotransferase (AST), albumin (ALB), alanine aminotransferase (ALT), creatine kinase (CK), creatinine (CREA), and urea (UREA) is performed using collected serum, and the result shows that all the parameters are within the reference range, as shown in FIG. 17. FIG. 17 is a diagram of serum testing of the animal model after treatment with the LMP whole tumor cell vaccine obtained according to Example 1 of the present disclosure and the cells in Comparative Examples. As shown in FIG. 17, the toxicity of the LMP whole tumor cell vaccine meets the requirements, and the LMP whole tumor cell vaccine is safe and biocompatible.

In the present disclosure, plant polyphenols and metal ions form dense nanocoating with non-covalent bonds (complex bonds) on the surface of tumors rapidly at room temperature. The embodiments and claims show that vortex mixing for about 10 seconds achieves complete formation, demonstrating exceptionally fast assembly kinetics. The physical-based single-cell modification process forms a dense coating on individual tumor cells, resulting in immediate cellular inactivation. Existing tumor cell vaccine platforms are mainly based on lysed cells followed by protein reconstitution, cryo-inactivated intact cells, or pure water incubation-induced cellular inactivation. In the present disclosure, the tumor cells are inactivated by polyphenols and metal ions forming a coating at room temperature.

Dual-functional metal ions serve both structural and immunological roles. On one hand, the metal ions complex with plant polyphenols to form a nanocoating, and on the other hand, as immune adjuvants metal ions, the metal ions are endocytosed to the antigen-presenting cells and then released in the intracellular acidic environment as immune adjuvants that activates the STING signaling pathway of the immune system and stimulates type I interferon production. The strong polyphenol-protein interactions enable complete retention of tumor cell antigens, addressing the limitations of current tumor vaccines that rely on single/specific antigens or suffer from insufficient antigen expression.

The multiple interaction capabilities of plant polyphenols allow for the convenient incorporation of functional molecules, such as LPS coating, anti-PD-L1 or the like. Further modifications, such as LPS on nanocoating, can promote the endocytosis of the LMP whole tumor cell vaccine by the antigen-presenting cells (e.g., DCs), subsequently promoting the maturation of DCs and triggering powerful anti-tumor immunity. Due to the adhesiveness of TA, PD-L1 can be simply loaded onto cell surfaces via non-covalent bonds, combining cell therapy with checkpoint blockade immunotherapy.

The present disclosure describes a novel method for preparing tumor whole tumor cell vaccine that achieves rapid inactivation under mild conditions, complete preservation of tumor antigens, and effective loading of immune activators to enhance anti-tumor efficacy.

## Claims

1. A method for preparing metal-polyphenol nanocoating-encapsulated tumor cells, comprising:

   step (a): mixing a plant polyphenol solution and a tumor cell suspension to obtain a first mixture;
   step (b): adding a manganese ion-containing compound solution to the first mixture, and mixing to obtain a second mixture, wherein a mass ratio of the plant polyphenol to the manganese ion-containing compound is in a range of 1:2 to 1:8; and
   step (c): adding a buffer solution to the second mixture, mixing, washing, and centrifuging to obtain metal-polyphenol nanocoating-encapsulated tumor cells.

2. The method of claim 1, wherein the plant polyphenol is one of tannic acid, tara tannin, and (-)-epigallocatechin gallate (EGCG).

3. The method of claim 1, wherein the manganese ion-containing compound in the step (b) is manganese sulfate monohydrate.

4. The method of claim 1, wherein after the step (c), the method further comprises: mixing the metal-polyphenol nanocoating-encapsulated tumor cells with a lipopolysaccharide solution, washing, and centrifuging to obtain a lipopolysaccharide-manganese ion-plant polyphenol (LMP) whole tumor cell vaccine.

5. The method of claim 4, wherein the mixing in the step (a), the step (b), and the step (c) is performed by vortexing.

6. The method of claim 4, wherein the metal-polyphenol nanocoating-encapsulated tumor cells are first mixed with a binder, then mixed with a lipopolysaccharide, and a mass ratio of the binder to the plant polyphenol is in a range of 1:1 to 1:10.

7. The metal-polyphenol nanocoating-encapsulated tumor cells prepared according to the method of claim 1-6, wherein a plant polyphenol-manganese ion coating is constructed on a surface of the tumor cells.

8. The metal-polyphenol nanocoating-encapsulated tumor cells according to claim 7, wherein the tumor cells are melanoma cells, murine dendritic cell line 2.4(DC2.4 cells), or Raw264.7 cells.

9. The metal-polyphenol nanocoating-encapsulated tumor cells according to claim 8, wherein the metal-polyphenol nanocoating-encapsulated tumor cells are used to prepare a whole tumor cell vaccine.

10. An application of the metal-polyphenol nanocoating-encapsulated tumor cells according to claim 9 in preparation of an antitumor drug, comprising: mixing at least one of the metal-polyphenol nanocoating-encapsulated tumor cells or the whole tumor cell vaccine with a pharmaceutically acceptable adjuvant or other ingredients to obtain a pharmaceutically acceptable formulation.

FIG. 1

FIG. 2

Untreated tumor cell | LMP whole tumor cell vaccine

FIG. 3

LMP-Tara whole tumor cell vaccine | LMP-EGCG whole tumor cell vaccine

FIG. 4

FIG. 5

LMP whole tumor cell vaccine

Untreated whole tumor cell vaccine

FIG. 6

FIG. 7

FIG. 8

FIG. 9

FIG. 10

FIG. 11

Internalization of LMP vaccines by DC cells

FIG. 12

FIG. 13

FIG. 14

FIG. 15

FIG. 16

FIG. 17

## INTERNATIONAL SEARCH REPORT

| International application No. |
|---|
| **PCT/CN2024/141431** |

| A. | CLASSIFICATION OF SUBJECT MATTER |
|---|---|

A61K45/06(2006.01)i; A61K39/39(2006.01)i; A61K33/32(2006.01)i; A61K33/30(2006.01)i; A61K33/26(2006.01)i; A61P35/00(2006.01)i

According to International Patent Classification (IPC) or to both national classification and IPC

| B. | FIELDS SEARCHED |
|---|---|

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

WPABS, WPABSC, DWPI, CNTXT, ENTXT, ENTXTC, CNKI, Web of Science, Elsevier Science, 超星读秀, Chaoxing Duxiu: 多酚, 单宁酸, 鞣酸, 塔拉单宁, 茶多酚, 金属离子, 锰离子, 锌离子, 亚铁离子, 金属-多酚网络, 纳米, 涂层, 包裹, 包覆, 肿瘤, 全细胞, 疫苗, Polyphenol, Tannic acid, Tara tannin, Tea polyphenol, TA, Tara, EGCG, Mn2+, Zn2+, Fe2+, metal polyphenolic network, MPN, Nano, Coating, Encapsulating, Tumor, Whole Cell, Vaccine

| C. | DOCUMENTS CONSIDERED TO BE RELEVANT |
|---|---|

| Category* | Citation of document, with indication, where appropriate, of the relevant passages | Relevant to claim No. |
|---|---|---|
| Y | CN 115252773 A (HUNAN CHILDREN'S HOSPITAL et al.) 01 November 2022 (2022-11-01)<br>see claims 1-9, embodiment 1, and Experimental Example 2-5 | 1-7, 9-10 |
| Y | CN 112451677 A (CHINA PHARMACEUTICAL UNIVERSITY) 09 March 2021 (2021-03-09)<br>see claims 1-4 | 1-7, 9-10 |
| Y | KR 20230155047 A (RESEARCH & BUSINESS FOUNDATION SUNGKYUNKWAN UNIVERSITY) 10 November 2023 (2023-11-10)<br>see claims 1-7 | 1-7, 9-10 |
| Y | 胥芷灵等 (XU, Zhiling) et al. "金属-多酚网络在肿瘤诊疗中的研究进展 (Research Progress of Metal-polyphenol Network in Tumor Therapy)"<br>中国肿瘤 (China Cancer). Vol. 31, No. (6), 05 June 2022 (2022-06-05),<br>see abstract, table 1, and sections 2.2 and 4 | 1-7, 9-10 |

☑ Further documents are listed in the continuation of Box C.　☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **06 March 2025** | **15 March 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| | International application No. |
| --- | --- |
| | **PCT/CN2024/141431** |

### C. DOCUMENTS CONSIDERED TO BE RELEVANT

| Category* | Citation of document, with indication, where appropriate, of the relevant passages | Relevant to claim No. |
| --- | --- | --- |
| Y | 梁佳仪 (LIANG, Jiayi). "以肿瘤细胞为模版的多酚-铝配位微颗粒疫苗制备方法及肿瘤免疫治疗效果 (Non-official translation: Preparation Method for Polyphenol-Aluminum Coordination Microparticle Vaccine Using Tumor Cells as Template and Tumor Immunotherapy Effect)" CNKI中国优秀硕士学位论文全文数据库 (Chinese Master's Theses Full-text Database), 15 February 2020 (2020-02-15), see abstract | 1-7, 9-10 |

Form PCT/ISA/210 (second sheet) (July 2022)

**INTERNATIONAL SEARCH REPORT**

| International application No. |
| --- |
| **PCT/CN2024/141431** |

**Box No. II      Observations where certain claims were found unsearchable (Continuation of item 2 of first sheet)**

This international search report has not been established in respect of certain claims under Article 17(2)(a) for the following reasons:

1. [✓]  Claims Nos.: **8**
      because they relate to subject matter not required to be searched by this Authority, namely:

      The subject matter of claim 8 is a method for treating a human or animal body, which falls within subject
      matter for which no search is required as defined in PCT Rule 39.1(iv).

2. [ ]  Claims Nos.:
      because they relate to parts of the international application that do not comply with the prescribed requirements to such an
      extent that no meaningful international search can be carried out, specifically:

3. [ ]  Claims Nos.:
      because they are dependent claims and are not drafted in accordance with the second and third sentences of Rule 6.4(a).

Form PCT/ISA/210 (continuation of first sheet) (July 2022)

**INTERNATIONAL SEARCH REPORT**
Information on patent family members

International application No.

**PCT/CN2024/141431**

| Patent document cited in search report | | | Publication date (day/month/year) | Patent family member(s) | | | Publication date (day/month/year) |
|---|---|---|---|---|---|---|---|
| CN | 115252773 | A | 01 November 2022 | None | | | |
| CN | 112451677 | A | 09 March 2021 | None | | | |
| KR | 20230155047 | A | 10 November 2023 | KR | 102744418 | B1 | 18 December 2024 |

Form PCT/ISA/210 (patent family annex) (July 2022)

**EP 4 763 225 A1**

**REFERENCES CITED IN THE DESCRIPTION**

**Patent documents cited in the description**

- CN 201910810918

- CN 202011095900